# EUROPÄISCHE PATENTANMELDUNG

(11) **EP 1 803 349 A1**
(43) Veröffentlichungstag der Anmeldung: **04.07.2007**
(21) Anmeldenummer: 07008238.3
(22) Anmeldetag: 11.02.2005
(51) Int. Cl.: A01N 25/30

(54) **Verwendung von alkoxylierten Alkyl- und Alkenylpolyglykosiden**

(30) Priorität: 20.02.2004 DE 102004008302
(62) Teilanmeldung aus: 05707328.0
(71) Anmelder: Cognis IP Management GmbH, 40589 Düsseldorf (DE)
(72) Erfinder: Behler, Ansgar, Dr., 46240 Bottrop (DE); Clasen, Frank, 40721 Hilden (DE)
(74) Vertreter: Fabry, Bernd

(57) **Zusammenfassung**

Vorgeschlagen wird die Verwendung von alkoxylierten Alkyl- und/oder Alkenylpolyglykosiden als Adjuvants in agrochemischen Formulierungen, speziell als Wirkverstärker für Herbizide vom Glyphosat-Typ.

## Beschreibung

### Gebiet der Erfindung

Die Erfindung liegt auf dem Gebiet der Agrochemikalien und betrifft die Verwendung spezieller nichtionischer Tenside als Adjuvants in agrochemischen Formulierungen.

### Stand der Technik

Agrochemische Zubereitungen, die anionische Tenside enthalten sind aus dem Stand der Technik bekannt. So beschreibt die US 6,746,988 (Syngenta) Mittel, welche aktive Wirkstoffe (beispielsweise Pestizide oder Herbizide), Alkylpolyglykoside sowie ausgewählte anionische Tenside enthalten. Die genannten Alkylpolyglykoside können dabei auch Polyethergruppen enthalten, nämlich dann, wenn die Produkte durch Acetalisierung von Zuckern oder Stärken mit alkoxylierten Alkoholen erhalten wurden; es handelt sich dabei indes nicht um alkoxylierte Alk(en)ylpolyglykoside.

Die Aufgabe der vorliegenden Erfindung hat darin bestanden, neue Tenside mit hinreichenden emulgierenden Eigenschaften zur Verfügung zu stellen, die die Eigenschaften von Wirkstoffen, speziell von Glyphosat in synergistischer Weise verstärken.

### Beschreibung der Erfindung

Gegenstand der Erfindung ist die Verwendung von alkoxylierten Alkyl- und/oder Alkenylpolyglykosiden als Adjuvants in agrochemischen Formulierungen, speziell als Wirkverstärker für Herbizide.

Überraschenderweise wurde gefunden, dass sich alkoxylierte Alkyl- und/oder Alkenylpolyglykoside wegen ihrer nichtionischen Eigenschaften besonders leicht in agrochemische Formulierungen, speziell Emulsionen einarbeiten lassen, sondern auch die Eigenschaften von Herbiziden und Pestiziden, insbesondere von Glyphosat in synergistischer Weise verstärken.

### Alkyl- und/oder Alkenylpolyglykoside

Alkyl- und Alkenylpolyglykoside, die als Ausgangsstoffe für die erfindungsgemäß zu verwendenden Alkoxylierungsprodukte dienen, stellen bekannte nichtionische Tenside dar, die der Formel (I) folgen,

**R¹O-[G]ₚ** **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenylpolyglykoside können sich von Aldosen bzw. Ketosen mit 5 oder 6 Kohlenstoffatomen, vorzugsweise der Glucose ableiten. Die bevorzugten Alkyl- und/oder Alkenylpolyglykoside sind somit Alkyl- und/oder Alkenylpoly**glucoside**. Die Indexzahl p in der allgemeinen Formel (**I**) gibt den Polymerisierungsgrad (DP), d. h. die Verteilung von Mono- und Polyglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkylpolyglykosid eine analytisch ermittelte rechnerische Größe, die meistens eine gebrochene Zahl darstellt. Vorzugsweise werden Alkyl- und/oder Alkenylpolyglykoside mit einem mittleren Polymerisierungsgrad p von 1,1 bis 3,0 eingesetzt. Aus anwendungstechnischer Sicht sind solche Alkyl- und/oder Alkenylpolyglykoside bevorzugt, deren Polymerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkylpolyglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkylpolyglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkylpolyglucoside auf Basis von gehärtetem C_{12/14}-Kokosalkohol mit einem DP von 1,1 bis 3.

### Herstellung der alkoxylierten Alkyl- und/oder Alkenylpolyglykoside

Die Anlagerung von Alkylenoxide an die freien Hydroxylgruppen von Alkyl- und/oder Alkenylpolyglykosiden ist aus dem Stand der Technik hinlänglich bekannt. Gemäß der US 3,737,426 erfolgt nach der Umsetzung von Stärke mit Ethylenglykol anschließend eine Alkoxylierung mit Ethylenoxid und/oder Propylenoxid bei etwa 170 °C unter Ausschluss von Wasser. Die Druckschrift US 3,640,998 empfiehlt die Alkoxylierung bei 100 bis 200 °C und unter Einsatz von basischen Katalysatoren durchzuführen, um die Zersetzung von Stärke zu minimieren. Es handelt sich hier um eine Standardalkoxylierung, bei der stets Luftsauerstoff und Wasser vor der Alkoxylierung entfernt werden. Aus der Druckschrift US 4,834,903 sind zudem Reaktionsmischungen von alkoxylierten Alkylmono- und Alkylpolyglykosiden bekannt, die hergestellt werden durch Alkoxylierung bei 120 bis 170 °C, unter Einsatz eines basischen Katalysators und unter im wesentlichen wasserfreien Bedingungen. In der besagten Schrift wird ausdrücklich herausgestellt, dass der Wassergehalt der Reaktionsmischung unter 5, vorzugsweise unter 1 Gew.-% liegen muss.

Für das eigentliche Alkoxylierungsverfahren hat es sich als vorteilhaft erwiesen, die Alkyl- und/oder Alkenylpolyglykoside der Formel (I) in Form ihrer wässrigen Zubereitungen in einem Druckreaktor mit Rührer vorzulegen, den Katalysator zuzusetzen und den Autoklav vor der Reaktion gründlich mit Stickstoff zu spülen, um alle Spuren von Luftsauerstoff zu entfernen. Danach empfiehlt es sich, den Druckbehälter aufzuheizen, wobei die Alkoxylierung vorzugsweise bei Temperaturen im Bereich von 80 bis 150 und insbesondere bei 100 bis 120°C durchgeführt wird. Das Alkylenoxid, bei dem es sich um Ethylenoxid, Propylenoxid, Butylenoxid oder Mischungen hiervon handeln kann, wird vorzugsweise über einen Heber in den Reaktor eingepresst, wobei der autogene Druck bis auf maximal etwa 5 bar ansteigen kann. Vorzugsweise werden pro Mol Alkyl- und/oder Alkenylpolyglykosid durchschnittlich 0,5 bis 100, bevorzugt 0,5 bis 20 und insbesondere 1 bis 15 Mol Alkylenoxid, vorzugsweise Ethylenoxid, eingesetzt. Die Anlagerung des Alkylenoxids erfolgt dabei statistisch, d.h. bei der Alkoxyierung wird ein komplexes Gemisch unterschiedlich hoch alkoxylierter Alkyl- und/oder Alkenylpolyglykoside erhalten. Das Ende der Reaktion ist daran zu erkennen, dass der Druck im Reaktor abfällt. In der Regel liegt die Reaktionszeit zwischen 30 Minuten und 2 Stunden. Aus Sicherheitsgründen empfiehlt es sich, die Mischung nachreagieren zu lassen, vorzugsweise bei den oben genannten Temperaturen, und anschließend noch weitere 30 min bei niederen Temperaturen bis etwa 80°C, ehe der Reaktor abgekühlt und entspannt wird.

Die Alkoxylierung erfolgt in Gegenwart von Katalysatoren, vorzugsweise basischen (alkalischen) Katalysatoren wie Natriummethanolat, Natriumhydroxid und/oder Kaliumhydroxid. Besonders bevorzugt sind Natriumhydroxid und Kaliumhydroxid, die in vorteilhafter Weise in Form wässriger Lösungen eingesetzt werden, die in der Regel 40 bis 60 gew.% ig an Katalysator sind. Zweckmäßig sind Katalysatormengen von 0,1 bis 5,0 Gew.%, vorzugsweise 0,2 bis 0,6 Gew.-% - berechnet als Feststoff und bezogen auf erhaltenes Reaktionsprodukt.

### Gewerbliche Anwendbarkeit

Unter agrochemischen Formulierungen, zu deren Herstellung die alkoxylierten Alkyl- und/oder Alkenylpolyglykoside im Sinne der Erfindung verwendet werden, sind im Folgenden alle Verbindungen zu verstehen, die Wirkstoffe aus der Gruppe der Fungizide, Düngemittel, Herbizide, Pestizide, Insektizide, Pflanzenstärkungsmittel oder andere Aktivsubstanzen zum Einsatz im Pflanzenbau enthalten. Besonders bevorzugt ist die Verwendung bei herbizidhaltigen Formulierungen.

Im Sinne der vorliegenden Erfindung werden die alkoxylierten Alkyl- und/oder Alkenylpolyglykosiden als Adjuvants, insbesondere als Wirkverstärker, verwendet. Besonders herausragende Wirkungsverstärkung wird für Glyphosat beobachtet. In der Regel sind die alkoxylierten Alkyl- und/oder Alkenylpolyglykoside und die Wirkstoffe in den agrochemischen Formulierungen in Gewichtsverhältnissen von 1:40 bis 3:1, vorzugsweise von 1:20 bis 1:1 1 enthalten.

Bei Glyphosat handelt es sich um N-(Phosphonomethyl)glycin, C₃H₈NO₅P, MG 169,07, Schmelzpunkt 200 °C, LD₅₀ (Ratte oral) 4320 mg/kg (WHO), ein nicht-selektives systemisches Blatt-Herbizid, das vorzugsweise in Form seines Isopropylamin-Salzes zur totalen und semitotalen Bekämpfung von Ungräsern und Unkräutern, einschließlich tiefwurzelnder mehrjähriger Arten, auf allen Ackerbaukulturen, im Obst- u. Weinbau verwendet wird. Die Struktur ist wie folgt:

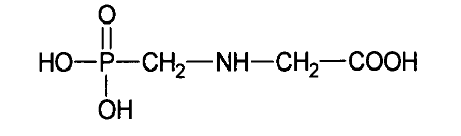

Unter Glyphosat werden alle dem Fachmann bekannten Derivate des Glyphosats verstanden, also vorzugsweise dessen Mono- oder Diethanolaminsalze des Glyphosats. Als Kationen kommen weiterhin Natrium oder Kalium in Frage. Besonders bedeutend ist das Isopropylaminsalz des Glyphosats. Weiterhin können auch beliebige Mischungen dieser Verbindungen im Rahmen der erfindungsgemäßen Verwendung eingesetzt werden.

Bei den Pestiziden, die auch in den agrochemischen Formulierungen enthalten sein können, handelt es sich vorzugsweise um öllösliche Substanzen. Es können dabei Fungizide, Herbizide, Insektizide oder deren Gemische zum Einsatz gelangen. Typische Beispiele für geeignete **Fungizide** sind Azoxystrobin, Benalaxyl, Carbendazim, Chlorothalonil, Cupfer, Cymoxanil, Cyproconazol, Diphenoconazol, Dinocap, Epoxiconazol, Fluazinam, Flusilazol, Flutriafol, Folpel, Fosetyl-Aluminium, Kresoxim-Methyl, Hexaconazol, Mancozeb, Metalaxyl, Metconazol, Myclobutanil, Ofurace, Phentinhydroxid, Prochloraz, Pyremethanil, Soufre, Tebucanazol, und Tetraconazol sowie deren Gemische. Als **Herbizide** können Alachlor, Acloniphen, Acetochlor, Amidosulfuron, Aminotriazol, Atrazin, Bentazon, Biphenox, Bromoxyl Octanoate, Bromoxynil, Clethodim, Chlodinafop-Propargyl, Chloridazon, Chlorsulfuron, Chlortoluron, Clomazon, Cycloxydim, Desmedipham, Dicamba, Dicyclofop-Methyl, Diharnstoff, Difluphenicanil, Dimithenamid, Ethofumesat, Fluazifop, Fluazifop-p-butyl, Fluorochloridon, Fluroxypyr, Glufosinat, Glyphosat, Haloxyfop-R, Ioxynil Octanoate, Isoproturon, Isoxaben, Metamitron, Metazachlor, Metolachlor, Metsulfuron-Methyl, Nicosulfuron, Notflurazon, Oryzalin, Oxadiazon, Oxyfluorphen, Paraquat, Pendimethalin, Phenmedipham, Phenoxyprop-p-Ethyl, Propaquizafop, Prosulfocarb, Quizalofop, Sulcotrion, Sulphosat, Terbutylazin, Triasulfuron, Trichlorpyr, Triflualin und Triflusulforon-Methyl einzeln oder in Abmischung eingesetzt werden. Als **Insektizide** kommen schließlich Biphenthrin, Carbofuran, Carbosulfan, Chlorpyriphos-Methyl, Chlorpyriphos-Ethyl, β-Cyfluthrin, λ-Cyhalothrin, Cyhexatin, Cypermethrin, Dicofol, Endosulfan, τ-Fluvalinat, α-Methrin, δ-Methrin, Phenbutatin, Pyrimicarb, Terbuphos und Tebuphenpyrad sowie deren Gemische in Betracht.

Falls gewünscht können die agrochemischen Formulierungen weitere übliche Hilfs- und Zusatzstoffe enthalten. Als fakultative Bestandteile können auch weitere Adjuvantien enthalten sein. Hierfür kommen beispielsweise nichtionogene Tenside aus mindestens einer der folgenden Gruppen in Frage:
(1) Anlagerungsprodukte von 2 bis 120 Mol Ethylenoxid und/ oder 0 bis 75 Mol Propylenoxid an lineare Fettalkohole mit 8 bis 22 C-Atomen, Fettamine, an Fettsäuren mit 8 bis 22 C-Atomen, an Alkylphenole mit 8 bis 15 C-Atomen in der Alkylgruppe und Fettaminen mit 6 bis 22 Kohlenstoffatomen;
(2) C_{12/18}-Fettsäuremono-, -di und -triester von Anlagerungsprodukten von 1 bis 120 Mol Ethylenoxid an Glycerin oder technische Oligoglycerine;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglyce-rinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

Insbesondere dann, wenn Pestizide in die Emulsionen eingearbeitet werden sollen, die bei Raumtemperatur Feststoffe darstellen, empfiehlt es sich, unpolare Lösemittel mit zu verwenden. Als weitere fakultative Komponenten kommen hierfür beispielsweise Mineralöle, Alkylaromaten und Kohlenwasserstoffe, wie sie unter der Bezeichnung Solvesso® von der Firma Exxon vertrieben werden, Fettsäureniedrigalkylester, wie z.B. die C₁-C₄-, d.h. die Methyl-, Ethyl-, Propyl- und/oder Butylester von Capronsäure, Caprylsäure, 2-Ethylhexansäure, Caprinsäure, Laurinsäure, Isotridecansäure; Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen in Frage. Des Weiteren geeignet sind pflanzliche Triglyceride, wie beispielsweise Kokosöl, Palmöl, Palmkernöl, Sonnenblumenöl, Olivenöl und dergleichen. Auch Polyethylenglykol ist ein geeignetes Lösemittel, vorzugsweise mit Molgewichten im Bereich von 90 bis 600 und vorzugsweise von 120 bis 250.

In den agrochemischen Formulierungen liegt der Wassergehalt in der Regel bei durchschnittlich 10 bis 90 und insbesondere 30 bis 60 Gew.-%. Die Applikationslösung für den eigentlichen Gebrauch enthält den eigentlichen Wirkstoff in Mengen von 0,01 bis 5 Gew.-%, vorzugsweise 0,1 bis 2,5 Gew.-% und insbesondere 0,1 bis 1,5 Gew.-%.

Die agrochemischen Formulierungen können aber auch als Konzentrate, beispielsweise mit 10 bis 90 Gew.-% Wirkstoff vertrieben werden, wobei erst vor der Anwendung die eigentliche Einsatzkonzentration durch Verdünnen eingestellt wird. Der Wassergehalt in solchen Konzentraten liegt zwischen 1 und 30 Gew.-%.

Es wurde beobachtet, dass die Kombination von Glyphosat mit den alkoxylierten Alkyl- und/oder Alkenylpolyglykosiden die Wirkung erhöht, so dass die Einsatzkonzentrationen der Wirkstoffe verringert und somit die negativen Auswirkungen des Einsatzes solcher Wirkstoffe auf die Umwelt wirksam reduziert werden können.

Die erfindungsgemäß zu verwendenden alkoxylierten Alkyl- und/oder Alkenylpolyglykoside können in den genannten agrochemischen Zubereitungen in Mengen von 0,05 bis 40, vorzugsweise 0,5 bis 25 und insbesondere 2,5 bis 10 Gew.-% - bezogen auf den Aktivsubstanzgehalt - eingesetzt werden.

### Beispiele

### Herstellbeispiel H1

### Kokosalkylpolyglykosid + 5 EO

In einem 1-1-Rührautoklaven wurden 697,0 g (0,8 Mol) C12/18-Kokosalkylpolyglykosid (Plantacare^{®} 1200, Cognis Deutschland GmbH & Co. KG) zusammen mit 4,7 g (entsprechend 0,67 Gew.% bezogen auf Ausgangsverbindung) einer wässrigen 50 Gew.%-igen Kaliumhydroxid-Lösung in einem Druckbehälter vorgelegt. Der Autoklav wurde verschlossen und dreimal abwechselnd mit Stickstoff gespült. Anschließend wurde bei einer Temperatur von maximal 120 °C und einem Druck von maximal 5 bar portionsweise 178,4 g (4,0 Mol) Ethylenoxid eindosiert. Die Reaktionszeit betrug 1 Stunde. Nach Beendigung der Ethoxylierung wurde 1 Stunde bei 120 °C nachreagiert und 30 Minuten bei 80 °C die Apparatur evakuiert, um Reste an nicht umgesetztem Ethylenoxid zu entfernen. Der Umsatz an Alkylpolyglykosid betrug 91,9 % der Theorie, der Polyethylenglykolgehalt lag unter 0,1 Gew.-%, der Feststoffgehalt bei 65,7 Gew.-%.

### Herstellbeispiel H2

### Kokosalkylpolyglykosid + 10 EO

In einem 1-1-Rührautoklaven wurden 568,0 g (0,69 Mol) Kokosalkylpolyglykosid zusammen mit 4,7 g (entsprechend 0,67 Gew.-% bezogen auf Ausgangsverbindung) einer wässrigen 50 Gew.%-igen Kaliumhydroxid-Lösung in einem Druckbehälter vorgelegt. Der Autoklav wurde verschlossen und dreimal abwechselnd mit Stickstoff gespült. Anschließend wurde bei einer Temperatur von maximal 120 °C und einem Druck von maximal 5 bar portionsweise 304,0 g (6,9 Mol) Ethylenoxid eindosiert. Die Reaktionszeit betrug 1 Stunde und 25 Minuten. Nach Beendigung der Ethoxylierung wurde 1 Stunde bei 120 °C nachreagiert und 30 Minuten bei 80 °C die Apparatur evakuiert, um Reste an nicht umgesetztem Ethylenoxid zu entfernen. Der Umsatz an Alkylpolyglykosid betrug 97,8 % der Theorie, der Polyethylenglykolgehalt lag unter 0,1 Gew.-%, der Feststoffgehalt bei 75,3 Gew.-%.

## Patentansprüche

1. Verwendung von alkoxylierten Alkyl- und/oder Alkenylpolyglykosiden als Adjuvants in agrochemischen Formulierungen.

2. Verwendung von alkoxylierten Alkyl- und/oder Alkenylpolyglykosiden als Wirkverstärker für Herbizide.

3. Verwendung nach Anspruch 2, **dadurch gekennzeichnet, dass** man als Herbizid Glyphosat einsetzt.

4. Verwendung nach mindestens einem der Ansprüche 1 bis 3, **dadurch gekennzeichnet, dass** man die alkoxylierten Alkyl- und/oder Alkenylpolyglykosiden durch Umsetzung von Alkylenoxiden mit Alkyl- und Alkenylpolyglykosiden der Formel (**I**),
**R¹O-[G]ₚ** **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, in Form von wässrigen Zubereitungen mit Wassergehalten über 5 Gew.-%. - berechnet als wässrige Zubereitung - erhält.

5. Verwendung nach mindestens einem der Ansprüche 1 bis 4, **dadurch gekennzeichnet, dass** man alkoxylierte Alkyl- und/oder Alkenylpolyglykoside der Formel (**I**) einsetzt, in der R¹ für einen Alkylrest mit 12 bis 14 Kohlenstoffatomen steht.

6. Verwendung nach mindestens einem der Ansprüche 1 bis 5, **dadurch gekennzeichnet, dass** man alkoxylierte Alkyl- und/oder Alkenylpolyglykoside der Formel (**I**) einsetzt, in der p für Zahlen von 1,1 bis 3 steht.

7. Verwendung nach mindestens einem der Ansprüche 1 bis 6, **dadurch gekennzeichnet, dass** man Alkyl- und/oder Alkenylpolyglykoside der Formel (**I**) einsetzt, in der G für einen Glucoserest steht.

8. Verwendung nach mindestens einem der Ansprüche 1 bis 7, **dadurch gekennzeichnet, dass** man alkoxylierte Alkyl- und/oder Alkenylpolyglykoside einsetzt, die pro Mol Alkyl- und/oder Alkenylpolyglykosid der Formel (**I**) 0,5 bis 100 Mol Alkylenoxid aufweisen.

9. Verwendung nach mindestens einem der Ansprüche 1 bis 8, **dadurch gekennzeichnet, dass** man ethoxylierte Alkyl- und/oder Alkenylpolyglykoside einsetzt.
